# EUROPEAN PATENT APPLICATION

(11) **EP 2 031 060 A1**
(43) Date of publication of application: **04.03.2009**
(21) Application number: 07075751.3
(22) Date of filing: 03.09.2007
(51) Int. Cl.: C12N 15/11, A61K 31/713

(54) **siDNA against influenza virus**

(71) Applicant: Mölling, Karin, 14195 Berlin (DE)
(72) Inventor: Mölling, Karin, 14195 Berlin (DE)
(74) Representative: Boeckh, Tobias

(57) **Abstract**

Silencing of Influenza virus RNA can be achieved by siDNA. These are oligodeoxynucleotides consisting of an antisense-strand homologous to the viral RNA and a second strand, partially complementary to the antisense-strand. The two strands are preferentially linked by a linker (eg 4 thymidines). Triple-helix formation with the target RNA is a preferred effect. The siDNA is superior to siRNA because it acts earlier, is easier taken up by the cell, the formation of RNA-DNA hybrids is preferred over double-stranded DNA or double-stranded RNA, which forms as tertiary structures in RNA genomes. Also the induction of interferon is less likely. siDNA is easier to synthesize and it is more stable. It can be combined with siRNA.

## Description

Silencing of Influenza Virus RNA can be achieved by siDNA. These are oligodeoxynucleotides consisting of an antisense-strand homologous to the viral RNA and a second strand, partially complementary to the antisense-strand. The two strands are preferentially linked by a linker (eg 4 thymidines). Triple-helix formation between siDNA and the target RNA is a preferred effect. The siDNA is superior to siRNA because the formation of RNA-DNA hybrids is preferred over double-stranded DNA or double-stranded RNA, which forms as tertiary structure in RNA genomes. Also the induction of interferon is less likely. Furthermore, siDNA is effective at earlier time-points after addition to the cell than siRNA. Therefore viral RNA is targeted by siDNA before it is amplified, while siRNA targets the about 1000fold higher viral RNA population of the progeny. siDNA is easier to synthesize and is more stable. It can be combined with other siDNAs targeted against various strains in a cocktail; it can also be combined with siRNA to target early and late steps in viral replication. siDNA can be targeted to any virus strain, including the present avian pandemic strain.

Influenza virus is a member of the Orthomyxoviruses causing wide-spread infection in the human respiratory tract, but existing vaccines and drug therapy are of limited value. In a typical year 20% of the human population is afflicted by the virus, resulting in 40.000 deaths. In one of the most devastating human catastrophies in history, at least 20 million people died worldwide during the 1918 Influenza A virus pandemic. The threat of a new influenza pandemic persists because existing vaccines or therapies are of limited value. In elderly the efficacy of vaccination is only about 40%. The existing vaccines have to be redesigned every year, because of genetic variation of the viral antigens, the Haemagglutinin HA and the Neuraminidase N. Four antiviral drugs have been approved in the United States for treatment and/or prophylaxis of Influenza. However, their use is limited because of severe side effects and the possible emergence of resistant viruses.

The siDNA, the basis for his invention, is a partially double-stranded hairpin-loop structured DNA oligonucleotide of about 20 to 50 nucleotides in length, whereby the antisense strand is fully homologous to the target viral RNA and the second strand only partially homologous to the antisense strand, forming a hairpin-loop structure. It forms an RNA-DNA hybrid with the antisense strand targeting the viral RNA. The siDNA may form triple-helix with the target RNA. An RNA-DNA hybrid is thermodynamically favored over double-stranded DNA; therefore the siDNA interacting with the viral RNA is preferred over RNA-RNA interactions.

The siDNA is highly sequence-specific, length-dependent and induces cleavage (silencing) of a target RNA by an RNase H (Moelling et al, FEBS Letters, 580, 3545-3550 (2006)), which is a component in some viruses but also in the cell. RNases H removes RNA primers required for the initiation of DNA synthesis or DNA replication in the nucleus or mitochondria, but they are also present in the cytoplasm.

siDNA is related to siRNA, whereby the cleavage enzyme is highly related to the RNase H.

Recently RNA interference has been used, whereby a short double-stranded RNA, 21 to 25 nucleotides in length, siRNA, directs sequence-specific degradation of messenger mRNA. Synthetic siRNA confers transient interference of gene-expression in a sequence-specific manner. This has significant medical applications. Inhibition of Influenza virus replication by siRNA against the viral RNA encoding the nucleocapsid or one component of the polymerase, the RNA transcriptase (PA), holds promise as prophylaxis or therapy against influenza virus infection in humans (Ge et al, PNAS 100, 2718-1723 (2003)).

It was recently demonstrated by the inventor the use of siDNA targeted to the HIV genome. This activates the RNase H of the virus and leads to silencing of the viral RNA (Matzen et al, Nature Biotechnol. 25, 669-674 (2007)). The RNase H is a Hybrid-specific RNase which cleaves RNA in an RNA-DNA hybrid and was discovered by Moelling et al (Nature New Biology 234, 240-243 (1971)). The inventor also observed that cellular RNase H-like activities such as RNase H1 and RNase H2A, B, C and even Agonaute 2 contribute to siDNA-mediated silencing. Agonaute 2 (Ago2) is the enzyme known to induce siRNA-mediated silencing. The inventor showed that Ago2 is enzymatically related to RNases H and can induce siDNA-mediated silencing as well (Moelling et al. Cold Spring Harb. Symp. Quant. Biol. 71, 365-368 (2006), Small Regulatory RNAs).

Furthermore, it could be shown by the inventor that siDNA is able to induce silencing of an oncogenic retrovirus, the Spleen Focus-Forming Virus and prevent infection, cause delay of disease progression and lead to increased survival time (Nature Biotechnol. 25, 669-674 (2007)). Furthermore the inventor showed in several cases that siDNA is superior to a single-stranded antisense effect (Jendis et al. AIDS Research and Human Retroviruses 12, 1161-1168 (1996), AIDS Research and Human Retroviruses 14, 999 - 1005, (1998), Moelling et al, FEBS Letters, 580, 3545-3550 (2006), Matzen et al. Nature Biotechnol. 25, 669-674 (2007)).

Thus the effect of siDNA has been demonstrated in several cases.

The problem to be solved with the present invention is to present an effective antiviral therapeutic against Influenza virus infections by siDNA.

The problem of the invention is solved by the features of the independent claims.

Therefore, siDNA oligonucleotides, capable of binding to one or more RNA target regions of Influenza virus are claimed as antiviral therapeutic, whereby the siDNA oligonucleotides are consisting of an antisense-strand homologous to the viral RNA target and a second strand, partially complementary to the antisense-strand forming a hairpin-loop structure. The siDNA oligonucleotides correspond to one or more Influenza virus target regions of at least 20 nucleotides in length, whereby the antisense strand of siDNA is fully or almost fully homologous to the target Influenza viral RNA. Conserved target RNA regions are preferred. Further, the second strand of the siDNA oligonucleotides is connected to the antisense strand through a thymidine linker, preferred - but not exclusively - 4 nucleotides in length, and whereby further the second strand is partially complementary to the antisense-strand and may be able to form triple helices by non-Watson-Crick base pairing with the viral RNA target strand.

Object of the invention is the use of siDNA as antiviral therapeutic. These are oligodeoxynucleotides consisting of an antisense-strand homologous to the viral RNA and a second strand, partially complementary to the antisense-strand. The siDNA is targeted to a conserved region of Influenza RNA, 20 to 25 nucleotides or longer, which is fully or almost fully homologous to the target RNA. A second strand is connected to the antisense strand through a Thymidine linker, e.g. 4 nucleotides in length. The second strand is partially complementary to the antisense-strand and may be able to form triple helices by non-Watson-Crick base pairing.

Also a preferred embodiment of the invention is the use of a combination of two or three siDNAs as described herein as antiviral therapeutic capable of binding to RNA target regions of Influenza virus as pharmaceutical agent. Further preferred is the use of a cocktail of different siDNA oligonucleotides to target different Influenza virus variants.

Influenza virus in the meaning of this invention is not restricted to Influenza A virus, Influenza B virus or Influenza C virus, but also to any virulent human pathogens of Influenza types. Therefore, other Influenza viruses such as avian Influenza virus are also an object of the invention. The serotypes that have been confirmed in humans, ordered by the number of known human pandemic deaths, are:
- H1 N1 caused "Spanish Flu."
- H2N2 caused "Asian Flu."
- H3N2 caused "Hong Kong Flu."
- H5N1 is a pandemic threat in 2006-7 flu season.
- H7N7 has unusual zoonotic potential.
- H1 N2 is endemic in humans and pigs.
- H9N2, H7N2, H7N3, H10N7.

The siDNA will be applied to an infected cell or an infected individual with a transducing agent, whereby as transducing agent is selected from the following group: the virus itself, a replicating Influenza virus particle, which carries the siDNA into the cell during the process of infection, a liposome, transmembrane carriers, peptides.

The siDNA oligonucleotides according to the invention are preferred as antiviral therapeutic as part of a pharmaceutical composition comprising these siDNA oligonucleotides capable of binding to RNA target regions of Influenza virus or its variants.

The preferred target site on the viral RNA is highly or at least somehow conserved. Targeting a subunit of the transcriptase and its silencing would reduce viral RNA replication efficiently.

It is an unexpected unique embodiment of the invention that it acts early on the virus replication cycle and holds promise as a prevention of infection. It could be applied as nasal spray and prevent virus propagation in the body, the effect called viremia. This cannot be accomplished by siRNA.

siDNA can also be targeted to cellular mRNAs coding for proteins essential for virus replication and indirectly prevent Influenza virus replication.

### More detailed description of the invention

The invention is based on the cognition that a DNA strand has a thermodynamic preference to form an RNA-DNA hybrid over double-stranded DNA or double-stranded RNA. Thus the invention is based on the not expected result that siDNA is of high preference (in contrast to siRNA) to form RNA-DNA hybrids; siDNA is therefore of advantage and a preferred object of the invention. Furthermore siDNA acts against the newly infecting incoming viral RNA, 2 to 3 days before siRNA. Thus a virus infection can be prevented by siDNA only. In contrast thereto, interferon-induction is a risk for siRNA and reduced or absent for an RNA-DNA hybrid or double-stranded DNA. Furthermore, siDNA is preferred due to the fact that DNA is easier to synthesize and more stable. Also, it can be combined with siRNA.

The siDNA may also be a chimeric molecule, consisting of ribonucleotides and desoxyribonucleotides. However, the RNase H cleavage site needs to consist of a local RNA-DNA hybrid.

siDNA is also superior to a simple antisense Oligodeoxynucleotide, because it is more stable during uptake and inside the cell and therefore more effective. It acts earlier than antisense DNA, and can target incoming RNA, while antisense DNA targets mRNA (Jekerle, V. et. al. J. Pharm. Sci. 8, 516-527 (2005)).

It was shown before that siDNA is targeting viral RNA before replication while siRNA is effective only late during replication targeting the mRNA (Westerhout, E. M. ter Brake, O. and Berkhout, B. Retrovirology 3,57-65 (2006)).

The invention takes advantage of this effect in silencing of Influenza Virus RNA. Using siDNA oligonucleotides is significantly more effective early during infection in contrast to siRNA. An unexpected advantage in conjunction with the present invention of siDNA *versus* siRNA is the targeting of incoming viral RNA before viral replication while siRNA is effective only late during replication targeting the mRNA. At this stage the RNA is highly amplified (e.g.1000fold or more) while the incoming RNA consists of a single copy. Viral RNA is destroyed within about 8 to14 hours post infection by a 5- to 10fold reduction. At this time point the siRNA effect is almost undetectable. The siRNA needs to enter a multi-protein complex, RISC, to prepare the cleavage, a process which takes about 3 days. By then the virus has replicated and spread through the organism efficiently. After 30 to 40 hours the reduction of viral RNA is similar in both cases, amounting to about 3- to 4fold reduction.

The RNA silencing inside the cell is achieved by the cellular RNases H, such as RNase H1 or RNase H2a, b, c. Also the Ago2, the siRNA-inducing silencing enzyme, is RNase H-like. These enzymes are located mainly in the nucleus but also in the cytoplasm. They are mainly responsible for removing RNA primers during DNA replication in the cell. The siDNA will lead to silencing of the viral RNA during early stages of replication and/or mRNA during late stages of replication, which requires higher doses. Treatment is - not limited to - performed by topical application, cream, spray, drops or injection of siDNA into the blood stream or intraperitoneally. Depending on the stability of the compound the treatment can be repeated. Toxicity has not been detected in mouse studies (Matzen et al. Nature Biotech. 25,669-674(2007)).

As stated above, it could be shown by the inventor that siDNA is able to induce silencing of an oncogenic retrovirus, the Spleen Focus-Forming Virus and prevent infection, cause delay of disease progression and lead to increased survival time (Nature Biotechnology 25, 669-674 (2007)). The mechanism was due to the action of the viral RNase H. A contribution of cellular RNases H or related cellular enzymes is likely. New unpublished results show that a reporter plasmid can be targeted by a siDNA and that the gene expression is silenced by RNase H1 or RNase H2a. This was demonstrated by inactivation of these RNases H, which strongly reduces the silencing effects.

### Preferred embodiments of the invention are described as follows:

G-tetrade formation may be beneficial for stability, cellular uptake, reduced need for carrriers, consisting e.g. of GGXXGGXXXXGGG etc, whereby X represents other nucleotides than G, such as A, T or C. siDNA oligonucleotides containing the siDNA oligonucleotides according to the invention contain G clusters to allow tetrade or tetramer formation or tetra-helices by sequences such as GGXXGGXXGG, up to 6Gs and 6 or 7X, whereby X is A, T or C. The hairpin-loop structure may consist preferably of about 9 non-self-complementary sequences at the 3'- and 5'-ends, followed by 6base-pairing, 2non-pairing, 3pairing, 2non-pairing, 3pairing sequences, followed by 4Ts (see for proof of principle Moelling et al, FEBS Letters, 580, 3545-3550 (2006)).

Similar to siRNA a combination of two or three siDNAs may be targeted to different regions of the RNA genome or a cocktail may be designed to target different Influenza Virus variants. These include avian strains as e.g. the present avian pandemic strain H5N1. The preferred siDNA sequences are connected by a linker, preferably a thymidine linker, in particular a T4-linker.

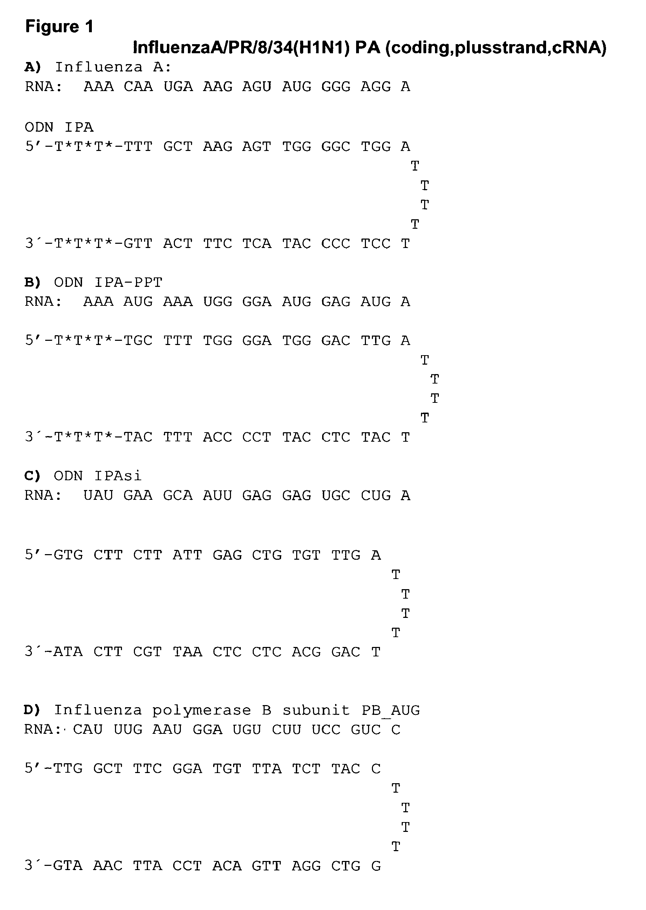

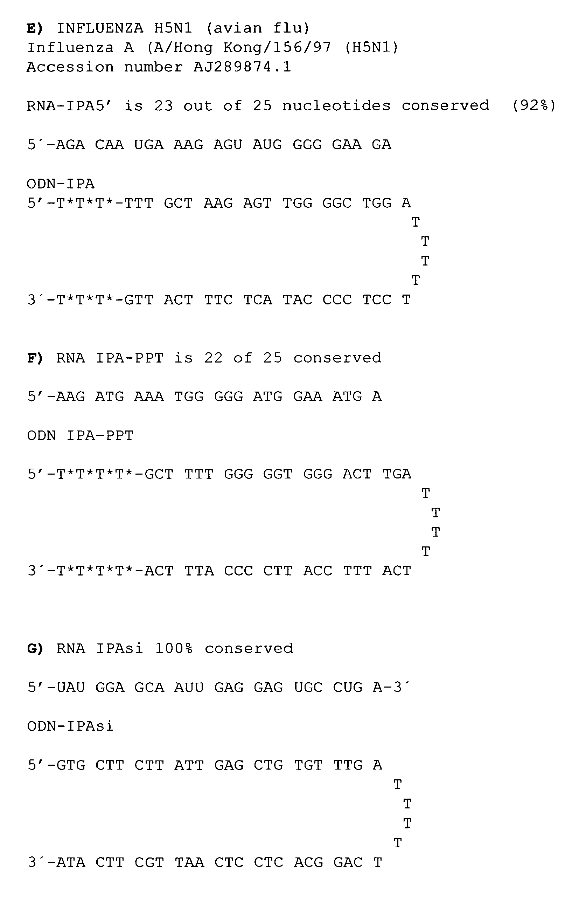

Preferred according to the invention are the sequences for siDNA as shown in figure 1, which are also an object of the invention:

| Sequence | Sequence name | Sequence |
|---|---|---|
| Seq.ID1 | Target Sequence (RNA) | 5'-AAACAAUGAAAGAGUAUGGGGAGG-3' |
| Seq.ID2 | ODN IPA5' (siDNA) - antisense | 5'-TTTTTTGCTAAGAGTTGGGGCTGGA-3' |
| Seq.ID3 | ODN IPA5' (siDNA) - second strand | 3'-TTTGTTACTTTCTCATACCCCTCCT-5' |
| Seq.ID4 | Target Sequence PPT (RNA) | 5'-AAAAUGAAAUGGGGAAUGGAGAUGA-3' |
| Seq.ID5 | ODN IPA-PPT (siDNA) - antisense | 5'-TTTTGCTTTTGGGGATGGGACTTGA-3' |
| Seq.ID6 | ODN IPA-PPT (siDNA) - second strand | 3'-TTTTACTTTACCCCTTACCTCTACT-5' |
| Seq.ID7 | Target Sequence si (RNA) | 5'-UAUGAAGCAAUUGAGGAGUGCCUGA-3' |
| Seq.ID8 | ODN IPAsi (siDNA)- antisense | 5'-GTGCTTCTTATTGAGCTGTGTTTGA-3' |
| Seq.ID9 | ODN IPAsi (siDNA)- second strand | 3'-ATACTTCGTTAACTCCTCACGGACT-5' |

The siDNA "antisense" strands and siDNA second strands are linked via a thymidine (T4) linker.

The siDNA may be stabilized by base modifications e.g. thioates at the ends and/or in the center. The invention is not restricted to those kinds of base modifications. Other base modifications, i.e phosphorothioates, P-DNA, sugar-phopsphate modifications, Morpholinos, amidates, 2'-OMe, 2'-F, 2'-MOE, LNA and further are also preferred without limitation to those modifications.

### Figures

**Abbreviations used are as follows:**

| | |
|---|---|
| ODN IPA 5': | Oligodeoxynucleotide Influenza A Polymerase A, 5' End |
| ODN IPA -PPT: | Polypurine-rich |
| ODN IPAsi: | target side used for siRNA |
| PB-AUG: | Polymerase B subunit. start site (AUG) |

Linkers are indicated as 4 Ts, or can also be symbolized by a curved line;
Linkers can also be modified by phosphothioate modifications
Stars (*) indicate phosphothioate-modifications

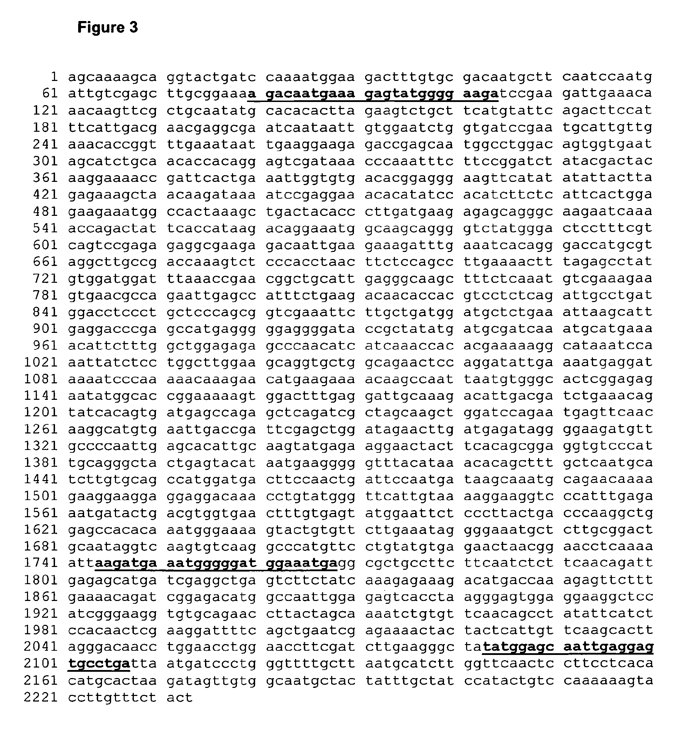

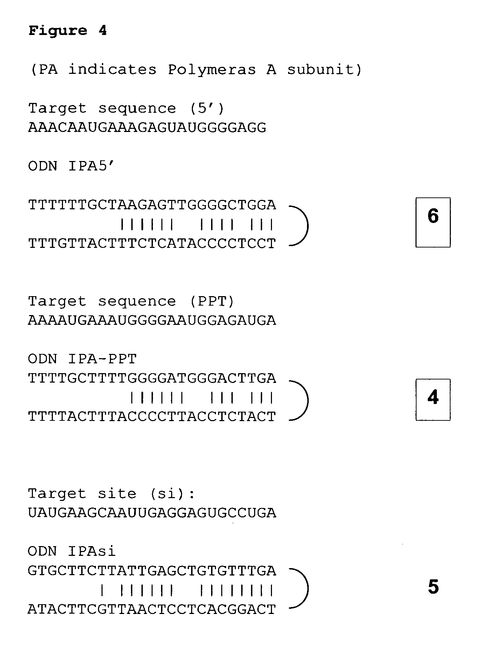

Vertical bars indicate Watson-Crick bonds
- Figure 1:: Preferred sequences according to the invention (A to G)

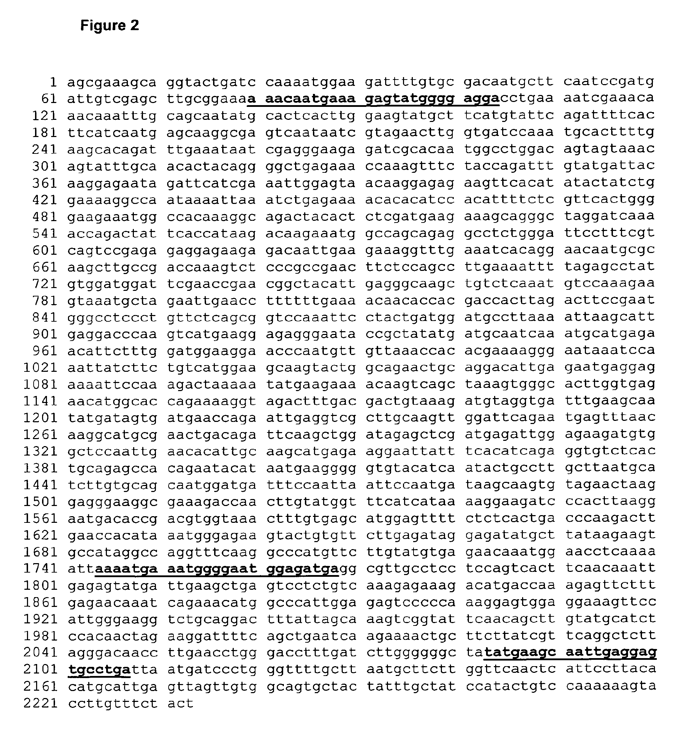

- Figure 2:: LOCUS EF467820 2233 bp cRNA linear VRL 11-MAR-2007 DEFINITION Influenza A virus (A/Puerto Rico/8/34(H1N1)) segment 3, polymerase PA,
complete sequence
underlined and bold are the targeted regions according to figure 1
- Figure 3:: Polymerase A (PA) of Influenza H5N1 (from Pubmed (nucleotides) (A/Hong kong/156/97 (H5N1)) Accession number AJ 289874.1
underlined and bold are the targeted regions according to figure 1
- Figure 4:: Preferred sequences according to the invention

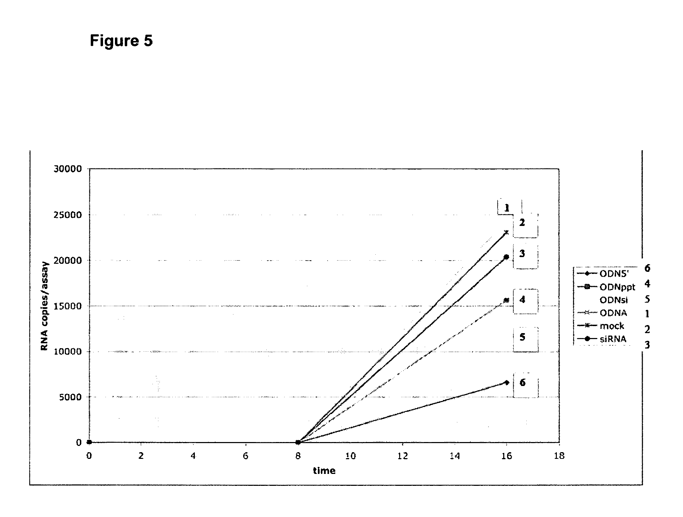

- Figure 5:: Influenza Virus was used to infect cells in tissue culture in the presence of ODNs against Influenza polymerase gene PA, three different regions (4, 5, 6, see figure 4). SiRNA was used as control and ODNA (FEBS.L. 580, 3545 (2006)) was used as an unspecific control. Lipofectamin was used to facilitate uptake. At the times indicated after infection the virus was determined in the supernatant by highly sensitive RT-PCR. Uninfected (mock-infected) cells served as another control.

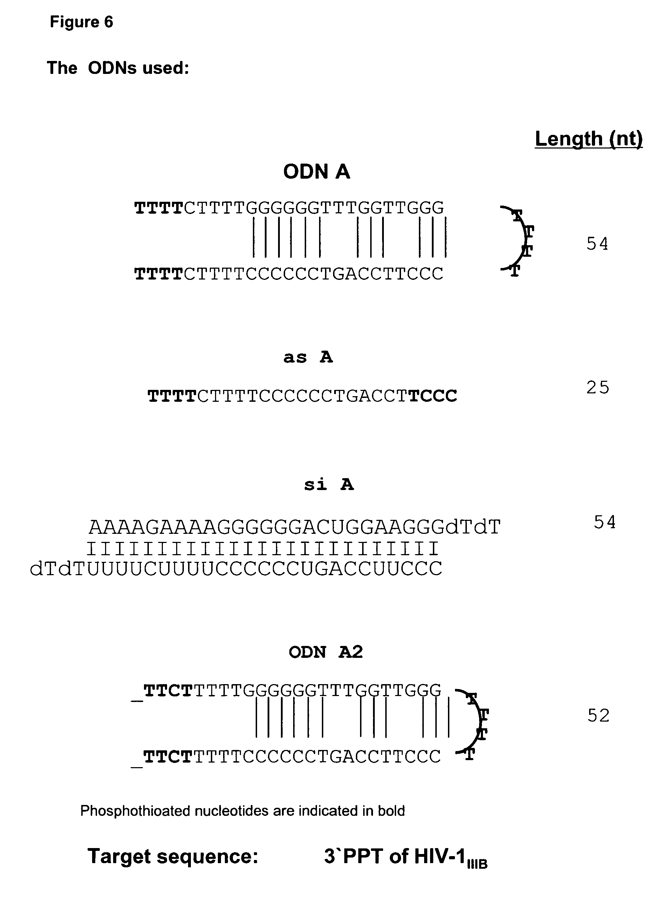

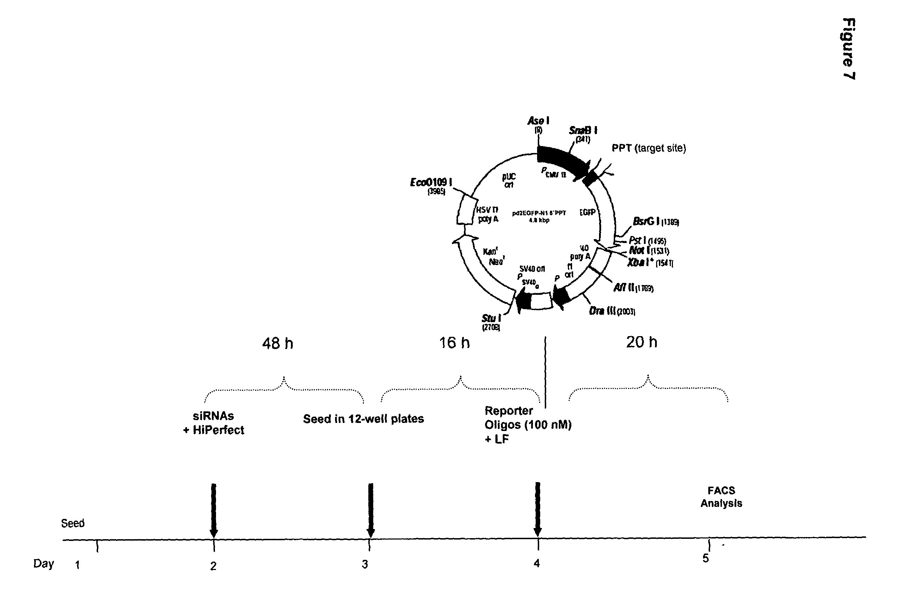

- Figure 6:: Sequences according to the experimental design of fig. 7
- Figure 7:: Determining the role of cellular RNase H1 and RNase H2A in the gene silencing effects of ODN A, asA and siA (see Fig. 3). Fig. 7 shows the experimantal design.

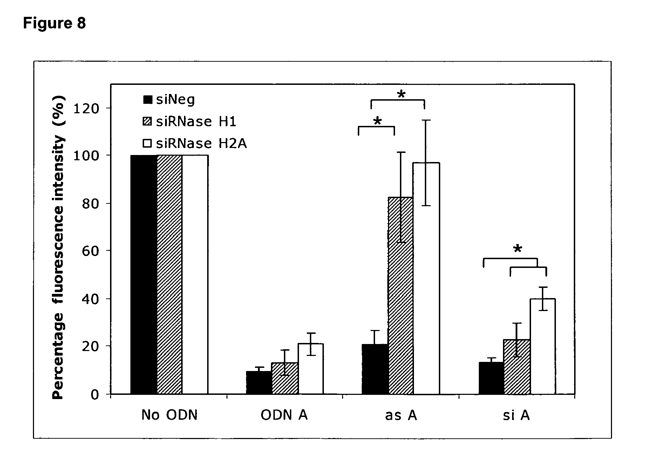

- Figure 8:: Knockdown of cellular RNase H1 or RNase H2A in HEK293 significantly reduced the gene silencing effects of asA but not those of ODN A or siA in live cells HEK293 cells were transfected with siNeg, siRNase H1 or siRNase H2A siRNA duplexes (2nM) with HiPerfect for 48 h, after which the cells were trypsinised and re-seeded into 12-well plates at a density of 5x105 cells per well. After overnight incubation at 37°C, the cells were co-transfected with pEGFP-5'PPT (360 ng or 113 fmole) and the various oligonucleotides (100 nM) using Lipofectamine2000 for 20 h before analysis by flow cytometry. The mean fluorescence intensities were normalized against those of control samples which were not treated with oligonucleotides.

## Claims

1. siDNA oligonucleotides, capable of binding to one or more RNA target regions of Influenza virus, as antiviral therapeutic, whereby the siDNA oligonucleotides are consisting of an antisense-strand homologous to the viral RNA target and a second strand, partially complementary to the antisense-strand.

2. siDNA oligonucleotides according to claim 1, whereby the siDNA molecule corresponds to one or more conserved Influenza target regions, of at least 20 nucleotides in length, whereby the antisense strand of siDNA is fully or almost fully homologous to the target Influenza viral RNA

3. siDNA oligonucleotides according to one or more of the foregoing claims, whereby the second strand is connected to the antisense strand through a thymidine linker, preferred 4 nucleotides in length, and whereby the second strand is partially complementary to the antisense-strand and may be able to form triple helices by non-Watson-Crick base pairing with the viral RNA target strand.

4. siDNA oligonucleotides preferably contain G clusters to allow tetrade or tetramer formation or tetra-helices or higher-ordered structures, facilitating cellular uptake, by sequences such as GGXXGGXXGG, up to 6Gs and 6 or 7X, whereby X is A, T or C .

5. siDNA oligonucleotides according to the preceding claims, whereby the siDNA is stabilized by base modifications.

6. Use of siDNA, or a combination of two or three siDNAs according to the preceding claims, as antiviral therapeutic, capable of binding to RNA target regions of Influenza or different Influenza virus variants, whereby different siDNA oligonucleotides may be combined in a cocktail as pharmaceutical agent.

7. Use according to claim 6, whereby the siDNA can also contain ribonucleotides, siDNA/RNA chimeras, or can be combined with siRNAs.

8. Use according to claim 6 or 7, whereby the siDNA will be applied to an infected cell or an infected individual with a transducing agent.

9. Use according to claim 8, whereby the transducing agent is selected from the following group: the virus itself, a replicating Influenza virus particle which carries the siDNA into the cell during the process of infection, a liposome, transmembrane carriers or peptides.

10. Pharmaceutical composition comprising siDNA capable of binding to RNA target regions of Influenza virus as antiviral therapeutic.
